# FASCICULE DE BREVET EUROPEEN

(11) **EP 0 549 483 B1**
(45) Date de publication et mention de la délivrance du brevet: **26.06.1996**
(21) Numéro de dépôt: 92420450.6
(22) Date de dépôt: 08.12.1992
(51) Int. Cl.: A61F 2/34

(54) **Noyau de cotyle pour prothèse de hanche**
Gelenkpfanne für Hüftprothese
Acetabular cup for hip prosthesis

(30) Priorité: 27.12.1991 FR 9116446
(43) Date de publication de la demande: 30.06.1993
(73) Titulaire: MERCK BIOMATERIAL FRANCE, 01800 Meximieux (FR)
(72) Inventeur: Caton, Jacques, F-69300 Caluire (FR); Picault, Charles, F-69110 Ste Foy Les Lyon (FR); Vidil, René, F-38700 La Tronche (FR); Prudhon, Jean-Louis, F-38330 Saint Ismier (FR); Michel, Claude-Régis, F-69370 St Didier au Mt d'Or (FR); Michel, Frédéric, F-69003 Lyon (FR); Collomb, Jean, F-26800 Porte Les Valence (FR); Majou, Claude, F-01800 Meximieux (FR)
(74) Mandataire: Laurent, Michel

(56) Documents cités:
- EP-A- 0 295 912
- FR-A- 2 597 747
- GB-A- 2 134 360
- US-A- 4 695 282

## Description

L'invention concerne un noyau destiné à être utilisé dans le cadre de la mise en place d'une prothèse de hanche.

Comme on le sait, une prothèse comprend fondamentalement deux parties, respectivement une tige destinée à être insérée dans le fémur à remplacer ou à renforcer, portant à son extrémité libre une tête sphérique venant s'articuler dans un cotyle, lui-même engagé dans la cavité cotyloidienne de l'aile illiaque. De manière traditionnelle, ce cotyle est généralement constitué de deux parties distinctes, à savoir respectivement :
- une cupule de soutien, destinée à être insérée dans la cavité cotyloïdienne de l'aile illiaque ;
- et un noyau ou insert, qualifié de "frottement", destiné à s'insérer dans la cupule de maintien, de coapter le plus possible à celle-ci, sa face interne de forme hémisphérique étant destinée à recevoir la tête sphérique de la tige de la prothèse.

On observe donc que de manière générale, la mise en place d'une prothèse implique l'utilisation d'une part, d'une cupule, et d'autre part, d'un noyau destiné à venir s'insérer dans la cupule.

Or, selon les cas pathologiques auxquels il est confronté, le praticien souhaiterait dans certains cas s'abstenir d'utiliser une cupule, et mettre directement en place le noyau. Dans d'autres cas en revanche, l'association cupule - noyau s'avère nécessaire. De la sorte, il souhaiterait pouvoir utiliser des inserts au noyau susceptibles d'être utilisés seuls, faisant dans ce cas directement office de cupule, soit en association avec une cupule traditionnelle.

L'objet de l'invention est de proposer un tel noyau. Selon l'invention, ce noyau de cotyle, destiné à recevoir la tête d'une prothèse de hanche, est en forme générale de coupole, et présente d'une part, une gorge annulaire au voisinage de sa base, gorge annulaire qualifiée d'équatoriale, et d'autre part, une gorge méridienne s'étendant le long d'un méridien, et joignant ladite gorge équatoriale en deux lieux diamétralement opposés.

Ce noyau se caractérise en ce que le noyau de cotyle comprend un élément annulaire constitué d'un jonc métallique comportant deux boucles perpendiculaires l'une par rapport à l'autre, une première boucle annulaire destinée à être reçue dans la gorge équatoriale, et une seconde boucle partielle, destinée à être reçue dans la gorge méridienne, les deux boucles étant solidaires l'une de l'autre et étant mises en place et maintenues dans celle-ci de par effet de rappel élastique.

En d'autres termes, l'invention consiste à proposer un noyau, de forme générale en coupole connue, mais comportant des éléments susceptibles de permettre soit un ancrage direct, notamment par cimentation dans la cavité cotyloïdienne de l'articulation à renforcer ou à remplacer, soit destinée à coopérer avec des organes prévus à cet effet dans un cotyle traditionnel.

On connait, par exemple par le document GB-A-2 134 360, un noyau pour prothèse de hanche, présentant également une gorge équatoriale et une gorge diamétrale, destinées à recevoir un repère opaque aux rayons X, et typiquement un repère métallique, constitué de deux demi boucles s'étendant selon deux plans perpendiculaires l'un par rapport à l'autre. De la sorte, ce repère permet l'identification du positionnement du noyau lors de la réalisation de noyau. En revanche, un tel noyau ne peut être fixé dans un cotyle traditionnel, compte tenu du caractère incomplet du repère, qui ne remplit pas la totalité de la gorge équatoriale, mais seulement la moitié de celle-ci.

Selon une forme avantageuse de l'invention, le noyau comporte également deux autres gorges méridiennes, symétriques l'une de l'autre par rapport à l'axe générateur de la coupole constitutive du noyau, non débouchantes au niveau de la partie supérieure du noyau. Ces deux gorges ont pour rôle de favoriser l'ancrage osseux et la cimentation du noyau, lorsque celui-à est utilisé sans cupule.

Selon une autre forme avantageuse de l'invention, le noyau comporte également une autre gorge annulaire, parallèle à la gorge annulaire équatoriale, mais située au voisinage de son sommet. Comme dans le cas précédent, cette gorge favorise l'ancrage osseux et la cimentation du noyau lorsqu'il est utilisé sans cupule.

L'invention concerne également un cotyle pour prothèse de hanche du type comprenant :
- une cupule en métal de soutien destinée à être positionnée dans la cavité cotyloïdienne de l'aile illiaque de l'articulation à renforcer ou à remplacer, et comportant une pluralité d'orifices traversants régulièrement répartis, destinés à permettre sa fixation dans ladite cavité cotyloïdienne,
- et un noyau de frottement destiné à être inséré dans la cupule de soutien, et à recevoir la tête de la prothèse de hanche.

Ce cotyle se caractérise en ce que le noyau est constitué par un noyau conforme à celui précédemment décrit, et en ce que la surface interne de la base de la cupule comporte des saillies destinées à coopérer avec la première boucle annulaire du noyau selon le principe de clipsage.

Selon une forme avantageuse de l'invention, la coopération saillies - boucle annulaire s'effectue au niveau d'encoches méridiennes ménagées au niveau de la base du noyau.

Selon une autre forme avantageuse de ce cotyle, la cupule comporte à sa base une jupe inférieure, destinée à coopérer avec la collerrette d'appui équatoriale constituant la base du noyau.

La manière dont l'invention peut être réalisée et les avantages qui en découlent ressortiront mieux de l'exemple de réalisation qui suit donné à titre indicatif et non limitatif à l'appui des figures annexées.

La figure 1 est une représentations schématique en perspective d'un noyau conforme à l'invention.

La figure 2 est une représentation vue du dessus du noyau de la figure 1.

La figure 3 est une représentation schématique en perspective d'une cupule conforme à l'invention.

La figure 4 est une coupe transversale de la figure 3, le noyau étant en place dans la cupule.

La figure 5 est une vue du dessous de la figure 4.

La figure 6 est une représentation schématique en perspective de l'organe à double boucle conforme à l'invention.

La figure 7 en est une vue du dessus.

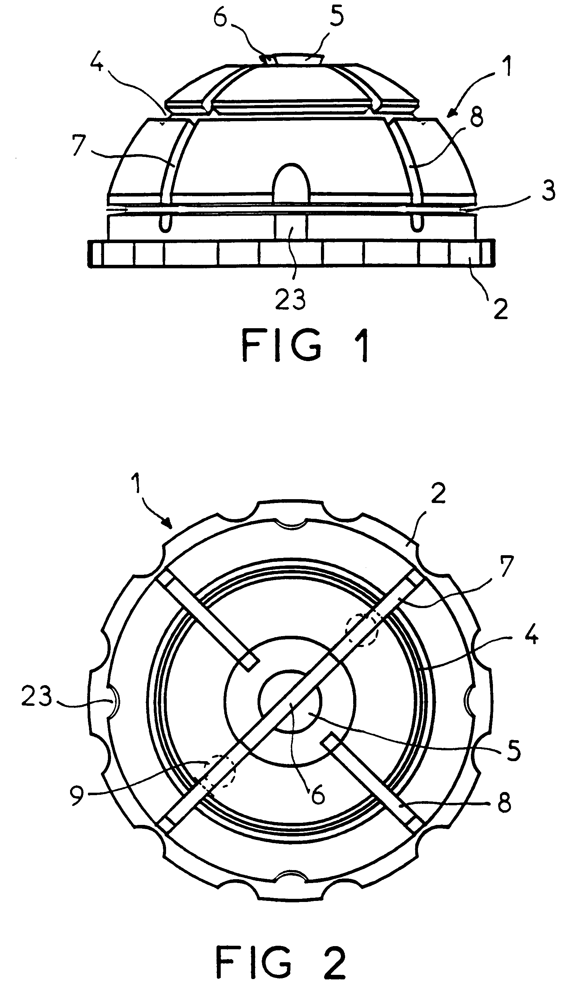

Selon l'invention et comme on peut le voir sur la figure 1, le noyau typiquement réalisé en polyéthylène haute densité, portant la référence générale (1), est en forme de coupole, dont l'extrémité supérieure est tronquée. La base (2) du noyau est constitué d'une collerette d'appui équatoriale, destinée, lorsque le noyau est inséré dans une cupule, à venir s'appuyer sur la base inférieure de celle-ci, comme par ailleurs il sera décrit plus en détail ultérieurement.

En outre, le noyau comporte une gorge annulaire (3), équatoriale, située juste en dessus de la collerette équatoriale (2). De cette gorge annulaire (3) part une autre gorge (7), méridienne, joignant deux points diamètralement opposés de ladite gorge annulaire (3), comme on peut le voir sur la figure 2.

De fait, cette gorge méridienne (7) comporte en fait deux parties de méridien opposées et symétriques l'une de l'autre par rapport à l'axe central (24) générateur de la coupole constitutive du noyau, et une partie relativement linéaire (6) au niveau de la partie supérieure tronquée du noyau. En outre, cette partie supérieure comporte un plot de positionnement (5), au niveau duquel traverse la gorge méridienne (7) selon la portion linéaire (6).

Le noyau comporte également une gorge annulaire (4), parallèle à la gorge équatoriale (3), mais située au voisinage de l'extrémité supérieure dudit noyau. Comme il sera décrit plus en détail ultérieurement, cette gorge (4) est destinée à favoriser la fixation et la repousse osseuse, lorsque le noyau est inséré directement au niveau de la cavité cotyloïdienne de l'articulation à renforcer ou à remplacer.

De plus, le noyau présente deux fentes méridiennes (8), symétriques l'une de l'autre par audit axe (24), non débouchantes tant au niveau de la gorges équatoriale (3), qu'à celui de la portion (6). De fait, et comme précédemment décrit, ces deux fentes (8) sont destinées à favoriser l'ancrage osseux du noyau, et partant sa fixation, lorsqu'il est inséré directement dans la cavité cotyloïdienne sans l'intermédiaire d'une cupule.

Enfin, la base dudit noyau comporte quatre encoches (23), séparées l'une de l'autre de 90°, débouchantes au niveau de la gorges équatoriale (3). Il sera également décrit ultérieurement le mode de fonctionnement de ces encoches et leur application.

L'ensemble de ces différents éléments, gorges (3,6,7), fentes (8), encoche (23), plot (5) sont directement taillés dans la masse. Dans une variante, le noyau peut être moulé, notamment par injection, ces différents éléments étant alors issus de moulage.

Enfin, afin de favoriser l'implantation du noyau, celui-ci comporte au niveau de sa base deux orifices (9), diamètralement opposés, et destinés à permettre le positionnement d'un ancillaire de mise en place.

Selon l'invention, ces orifices (9) sont situés dans le même plan que la gorge méridienne (7), comme on peut mieux le voir sur la figure 2.

De manière connue, ce noyau définit un volume hémisphérique interne (22), destiné à recevoir la tête de la prothèse de hanche.

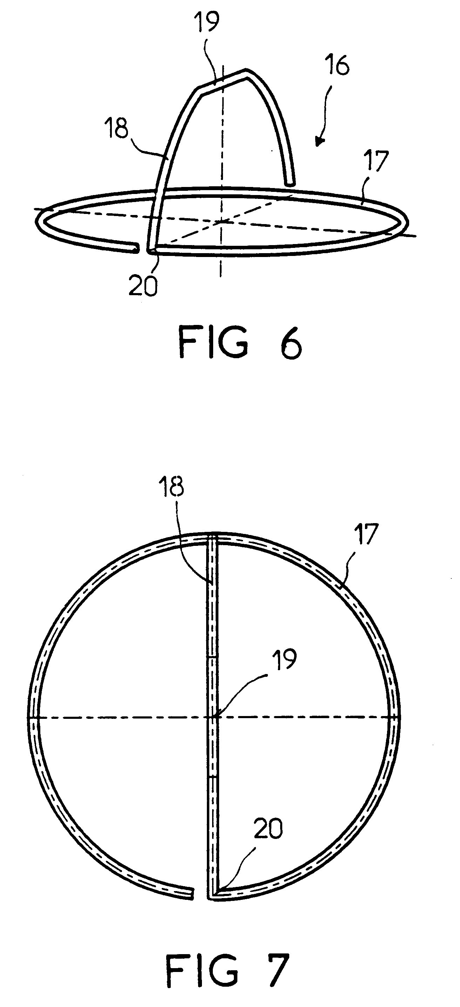

Ce noyau (1) est destiné à recevoir un élément annulaire (16), constitué d'un jonc métallique, typiquement réalisé en acier inoxydable, et susceptible d'être mis en place au niveau des gorges respectivement équatoriale (3) et méridienne (6,7) du noyau. De fait, cet élément annulaire (16) comporte une première boucle annulaire (17), venant se loger dans la gorge équatoriale (3) du noyau, et se poursuit par une seconde boucle partielle (18), destinée à être reçue dans la gorge méridienne (6,7), cette boucle (18) comportant une section linéaire (19), reçue dans la portion linéaire (6) de ladite gorge méridienne (7). Ces deux boucles (17) et (18) sont continues et sont issues du même jonc métallique et sont perpendiculaires l'une par rapport à l'autre, comme on peut le voir clairement dans la figure 6. Leur point de jonction s'effectue en (20). Cet élément annulaire (16) est réalisé de façon telle, que sa mise en place au niveau du noyau s'effectue en force et qu'elles nécessitent donc pour être ôter un outil particulier, à savoir un extracteur de noyau.

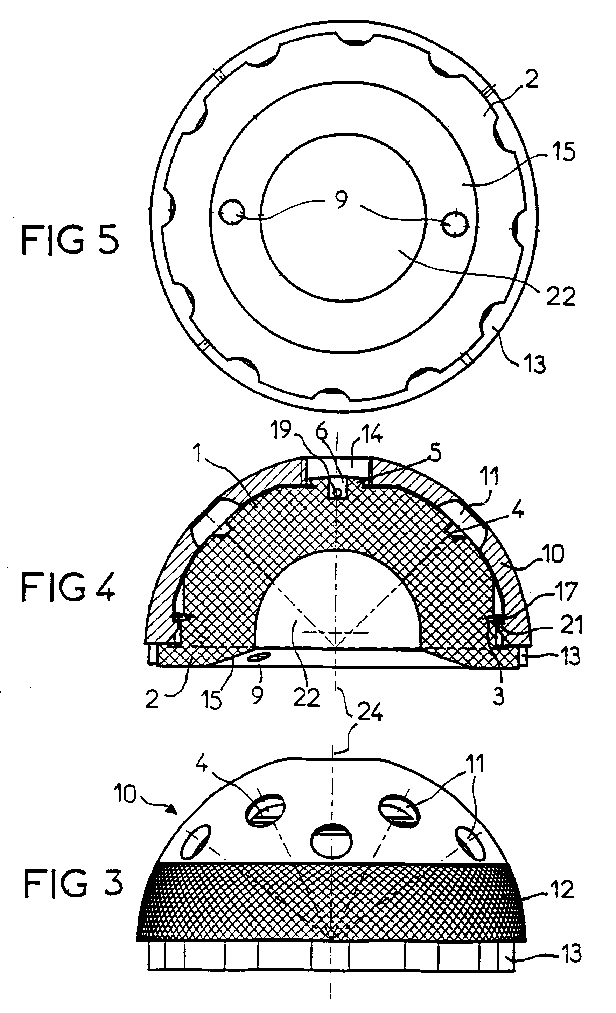

Cet élément annulaire (16) a une double fonction. Tout d'abord, il joue le rôle de radio-repère notamment, de par la seconde boucle partielle (18) et (19) mis en place au niveau de la gorge méridienne (6,7). En revanche, l'autre boucle (17) est destinée à permettre la coopération du noyau avec une cupule cotyloïdienne lorsque celle-ci est requise, et ce de manière connue. On a par exemple montré dans les figures 3 et 4, le noyau mis en place au niveau d'une cupule. La cupule (10) est de forme hémisphérique, et est réalisée en métal, notamment en acier inoxydable voire en alliage à base de titane. Sa face externe convexe se termine au niveau du pôle par une ouverture (14), destinée à permettre d'une part l'adaptation d'un outillage de pose dans la cavité cotyloïdienne et d'autre part, à recevoir le plot de positionnement (5) du noyau. Cette cupule représente de manière traditionnelle une symétrie de révolution. Elle comporte sur le tiers inférieur de sa hauteur des cannelures (12), usinées dans la masse, et destinées lorsque la cupule est en place dans la cavité cotyloïdienne à permettre une meilleure ossification au niveau des aspérités ainsi créées. Les deux autres tiers de la cupule comportent des orifices traversants et conformés (11), régulièrement réparties, et destinés à permettre le passage de vis de fixation d'une part, et d'autre part, l'escamotage des têtes de vis dans l'épaisseur de ladite cupule. Selon une autre caractéristique, la surface interne de la cupule comporte au niveau de sa base des saillies (21), destinées à coopérer avec les encoches (23) du noyau et la boucle primaire (17) de l'élément annulaire. Lorsqu'une telle cupule est requise pour la mise en place d'une prothèse de hanche, son positionnement s'effectue traditionnellement, sa fixation étant assurée au moyen de vis insérées dans les orifices (11). On met alors en place le noyau de frottement, muni de l'élément annulaire (16), permettant d'une part le repérage radio de celui-ci et d'autre part, le clipsage de la boucle primaire inférieure (17) au niveau des saillies (21) et des encoches (23).

De manière connue, le noyau est inséré à température ambiante et s'expanse à la température du corps humain jusqu'à aboutir à une coaptation maximum entre le noyau et la cupule, et ce, afin de diminuer le plus possible le fluage du polyéthylène. En outre, et de manière connue, cette coaptation maximum permet de transmettre le plus fidèlement possible les efforts entre la tête prothétique et la cupule. Ce noyau est avantageusement un chanfrein (15), destiné à augmenter le débattement de l'articulation et qui en outre permet de par son positionnement inférieur, d'éviter l'effet de came, qui de manière connue provoque une luxation de la hanche.

Lorsque la mise en place d'une cupule n'est pas nécessaire, on procède à la fixation du noyau directement au niveau de la cavité cotyloïdienne de l'aile illiaque par cimentation. Pour ce faire, le noyau comporte également l'élément annulaire (16), de sorte que l'on puisse toujours procéder à son repérage par rayons X. En outre, compte tenu de la présence d'une part, de la gorge annulaire supérieure (4) et d'autre part, des fentes méridiennes non débouchantes (8), l'ancrage osseux est favorisé, augmentant la rétention du noyau au sein de la cavité cotyloïdienne.

On conçoit de la sorte qu'avec un tel noyau universel, le praticien puisse en fonction de l'état de l'articulation qu'il doit renforcer ou remplacer, choisir le mode le plus approprié notamment au niveau du noyau ou l'association noyau-cupule, ce que les prothèses connues à ce jour ne permettaient pas d'obtenir. Ce choix permet de la sorte un meilleur traitement des problèmes liés à cette articulation.

## Revendications

1. Noyau de cotyle (1), destiné à recevoir la tête d'une prothèse de hanche, en forme générale de coupole, et présentant d'une part une gorge annulaire équatoriale (3) au voisinage de sa base (2), et d'autre part une gorge méridienne (6,7) s'étendant le long d'un méridien, et joignant ladite gorge équatoriale (3) en deux lieux diamètralement opposés, **caractérisé** en ce que le noyau de cotyle (1) comprend un élément annulaire (16) constitué d'un jonc métallique comportant deux boucles perpendiculaires l'une par rapport à l'autre, une première boucle annulaire (17), destinée à être reçue dans la gorge équatoriale (3), et une seconde boucle partielle (18,19), destinée à être reçue dans la gorge méridienne (6,7), les deux boucles étant solidaires (20) l'une de l'autre et étant mises en place et maintenues dans celle-ci de par effet de rappel élastique.

2. Noyau de cotyle selon la revendication 1, **caractérisé** en ce qu'il comporte deux fentes méridiennes (8), symétriques l'une de l'autre par rapport à l'axe générateur (24) de la coupole constitutive du noyau, non débouchantes au niveau de la partie supérieure (5,6) du noyau.

3. Noyau de cotyle selon l'une des revendications 1 et 2, **caractérisé** en ce qu'il comporte une autre gorge annulaire (4), parallèle à la gorge annulaire équatoriale (3), mais située au voisinage de son extrémité supérieure.

4. Cotyle pour prothèse de hanche, comprenant :
- une cupule en métal de soutien (10), destinée à être positionnée dans la cavité cotyloïdienne de l'aile illiaque de l'articulation à remplacer ou à renforcer, et comportant une pluralité d'orifices traversants (11) régulièrement répartis, destinés à permettre sa fixation dans ladite cavité cotyloïdienne,
- et un noyau de frottement (1) destiné à être inséré dans la cupule de soutien, et à recevoir la tête de la prothèse de hanche,
**caractérisé** en ce que le noyau (1) est constitué par un noyau conforme à l'une des revendications 1 à 3, et en ce que la surface interne de la base de la cupule (10) comporte des saillies (21), destinées à coopérer par clipsage avec la première boucle annulaire (17) du noyau (1).

5. Cotyle pour prothèse de hanche selon la revendication 4, **caractérisé** en ce que la coopération saillies (21) - boucle primaire annulaire (17) s'effectue au niveau d'encoches méridiennes (23) ménagées au niveau de la base du noyau.

6. Cotyle pour prothèse de hanche selon l'une des revendications 4 et 5, **caractérisé** en ce que la cupule (10) comporte à sa base une jupe inférieure (13), destinée à coopérer avec la collerrette d'appui équatoriale (2) constituant la base du noyau (1).

## Claims

1. Socket core (1), intended to receive the head of a hip prosthesis, of a general dome shape, and having on the one hand an equatorial annular throat (3) in the vicinity of its base (2), and on the other hand a meridional throat (6, 7) extending along a meridian and joining the said equatorial throat (3) at two diametrically-opposed points, characterised in that the socket core (1) comprises an annular member (16) made up of a metallic rod comprising two loops which are perpendicular one to the other, a first annular loop (17) being intended to be received in the equatorial throat (3), and a second, partial loop (18, 19), intended to be received in the meridional throat (6, 7), the two loops being integral (20) with one another and being positioned and retained in place by the effect of resilient return.

2. Socket core according to claim 1, characterised in that it comprises two meridional slots (8), symmetrical with one another with respect to the generating axis (24) of the dome constituting the core, and not opening at the level of the upper portion (5, 6) of the core.

3. Socket core according to one of claims 1 and 2, characterised in that it comprises another annular throat (4), parallel with the equatorial annular throat (3), but situated in the vicinity of its topmost point.

4. Socket for a hip prosthesis, comprising:
- a metallic supporting cup (10), intended to be positioned in the socket cavity of the iliac wing of the joint to be reinforced or replaced, and comprising a plurality of uniformly-distributed continuous openings (11), intended to enable it to be secured in the socket cavity,
- and a frictional core (1), intended to be inserted in the supporting cup and to receive the head of the hip prosthesis,
characterised in that
the core (1) is constituted by a core according to one of claims 1 to 3, and in which the internal surface of the base of the cup (10) comprises projections (21) intended to co-operate with the first annular loop (17) of the core (1), by means of snap engagement.

5. Socket for hip prosthesis according to claim 4, characterised in that the co-operation between projections (21) and primary annular loop (17) is produced at the level of meridional notches (23) produced at the level of the base of the core.

6. Socket for hip prosthesis according to one of claims 4 and 5, characterised in that the cup (10) has at its base a lower skirt (13) intended to co-operate with the equatorial support collar (2) constituting the base of the core (1).

## Patentansprüche

1. Gelenkpfanneneinsatz (1) zur Aufnahme des Kopfes einer Hüftprothese und im wesentlichen in Form einer Kuppel, welcher einerseits eine äquatoriale Ringnut (3) in der Nähe seiner Basis (2) und andererseits eine Meridiannut (6,7) aufweist, welche entlang eines Meridians verläuft und mit der genannten Äquatorialnut (3) an zwei diametral gegenüberliegenden Stellen zusammentrifft, **dadurch gekennzeichnet,** daß der Gelenkpfanneneinsatz (1) ein aus einer Metallspange bestehendes ringförmiges Element (16) aufweist, welches zwei rechtwinkling zueinander angeordnete Schlaufen aufweist, wobei eine erste, ringförmige Schlaufe (17) dazu bestimmt ist, in der Äquatorialnut (3) aufgenommen zu werden, und eine zweite, partielle Schlaufe (18,19) dazu bestimmt ist, in der Meridiannut (6,7) aufgenommen zu werden, wobei die beiden Schlaufen miteinander verbunden (20) sind und aufgrund ihrer elastischen Rückstellkraft in ihre Lage gebracht und dort gehalten werden.

2. Gelenkpfanneneinsatz nach Anspruch 1, **dadurch gekennzeichnet,** daß er zwei Meridiannuten (8) aufweist, die in bezug auf die Rotationsachse (24) der den Einsatz bildenden Kuppel zueinander symmetrisch sind und nicht in den oberen Teil (5,6) des Einsatzes münden.

3. Gelenkpfanneneinsatz nach einem der Ansprüche 1 und 2, **dadurch gekennzeichnet,** daß er in der Nähe seines oberen Endes eine weitere Ringnut (4) aufweist, welche parallel zu der äquatorialen Ringnut (3) verläuft.

4. Gelenkpfanne für Hüftprothesen, bestehend aus:
- einer Stützschale (10) aus Metall, die zur Anordnung in der gelenkpfannenförmigen Vertiefung des Hüftbeins des zu ersetzenden oder zu verstärkenden Gelenks bestimmt ist und eine Vielzahl von gleichmäßig verteilten durchgehenden Öffnungen (11) aufweist, welche die Befestigung der Schale in der genannten gelenkpfannenförmigen Vertiefung ermöglichen,
- und einem Reibungseinsatz (1), der zum Einfügen in die Stützschale und zum Aufnehmen des Kopfes der Hüftprothese bestimmt ist,
**dadurch gekennzeichnet,** daß der Einsatz (1) gemäß einem der Ansprüche 1 bis 3 ausgebildet ist und daß die Innenfläche der Basis der Schale (10) Vorsprünge (21) aufweist, welche dazu bestimmt sind, mit der ersten ringförmigen Schlaufe (17) des Einsatzes (1) rastend zusammenzuwirken.

5. Gelenkpfanne für Hüftprothesen nach Anspruch 4, **dadurch gekennzeichnet,** daß das Zusammenwirken der Vorsprünge (21) mit der ersten ringförmigen Schlaufe (17) im Bereich meridianer Ausnehmungen (23) erfolgt, welche im Bereich der Basis des Einsatzes angeordnet sind.

6. Gelenkpfanne für Hüftprothesen nach einem der Ansprüche 4 und 5, **dadurch gekennzeichnet,** daß die Schale (10) an ihrer Basis einen unteren Bund (13) aufweist, welcher mit dem äquatorialen Stützkragen (2), der die Basis des Einsatzes (1) bildet, zusammenwirkt.
